# EUROPEAN PATENT APPLICATION

(11) **EP 4 410 326 A1**
(43) Date of publication of application: **07.08.2024**
(21) Application number: 22883624.3
(22) Date of filing: 20.10.2022
(51) Int. Cl.: A61M 1/16

(54) **DIALYSIS SOLUTION EXTRACTION DEVICE AND BLOOD PURIFICATION DEVICE**

(30) Priority: 22.10.2021 JP 2021173501
(71) Applicant: Nikkiso Company Limited, Tokyo 150-6022 (JP)
(72) Inventor: SHIMOMURA, Yoshihiro, Chiba-shi, Chiba 263-8522 (JP); ASANO, Takuji, Tokyo 150-6022 (JP); TAKEZAWA, Azusa, Tokyo 150-6022 (JP); INOUE, Naoki, Tokyo 150-6022 (JP); SAPUTRA, Gabriel Pramudita, Tokyo 150-6022 (JP)
(74) Representative: von Hirschhausen, Helge
(86) International application number: PCT/JP2022/039048
(87) International publication number: WO 2023/068320

(57) **Abstract**

To provide a dialysate extraction device and a blood purification apparatus each configured such that the scattering of dialysate that may occur when a cap member is detached from a collecting part is prevented more assuredly. A dialysate extraction device includes a dialysate extraction port 14 including a collecting part 14a to which dialysate is collectable, a cap member 15 attachable to and detachable from the collecting part 14a of the dialysate extraction port 14, and a seal member 16 configured to seal the collecting part 14a with the cap member 15 attached to the collecting part 14a. The seal member 16 includes a close-fitting part 16a configured to seal the collecting part 14a by closely fitting on an outer peripheral surface of the dialysate extraction port 14, and a projection 16b projecting toward an inside of the collecting part 14a and having an outer peripheral surface 16ba that faces an inner peripheral surface 14aa of the collecting part 14a with a gap in between. The projection 16b defines an inflow route γ into which outside air is allowed to flow when the sealing by the close-fitting part 16a is undone.

## Description

### Technical Field

The present invention relates to a dialysate extraction device and a blood purification apparatus each including a cap member attachable to and detachable from a collecting part of a dialysate extraction port connected to a dialysate flow route, and a seal member configured to seal the collecting part with the cap member attached to the collecting part.

### Background Art

As disclosed by PTL 1, for example, a known dialysate extraction device includes a cap member attachable to and detachable from a collecting part of a dialysate extraction port connected to a dialysate flow route, and a seal member configured to seal the collecting part with the cap member attached to the collecting part. When the cap member is detached from the collecting part, the collecting part is opened to allow dialysate to be ejected to the outside.

### Citation List

### Patent Literature

PTL 1: Japanese Unexamined Patent Application Publication No. 2016-10477

### Summary of Invention

### Technical Problem

In the above known dialysate extraction device, the seal member is flexibly displaceable toward the collecting part so as to prevent the scattering of the dialysate that may occur when the cap member is detached. However, some fluid such as a cleaning solution occasionally remains strongly attracted to the tip of the dialysate extraction port with the tension thereof. When the cap member is detached in such a situation, remaining dialysate may scatter to the outside.

The present invention has been conceived in view of the above circumstances and provides a dialysate extraction device and a blood purification apparatus each configured such that the scattering of dialysate that may occur when a cap member is detached from a collecting part is prevented more assuredly.

### Solution to Problem

A dialysate extraction device according to an embodiment of the present invention includes a dialysate extraction port connected to a dialysate flow route through which dialysate is to flow, the dialysate extraction port including a collecting part to which the dialysate flowing in the dialysate flow route is collectable; a cap member attachable to and detachable from the collecting part of the dialysate extraction port; and a seal member attached to the cap member and configured to seal the collecting part with the cap member attached to the collecting part. The seal member includes a close-fitting part configured to seal the collecting part by closely fitting on an outer peripheral surface of the dialysate extraction port, and a projection projecting toward an inside of the collecting part and having an outer peripheral surface that faces an inner peripheral surface of the collecting part with a gap in between. The projection defines an inflow route into which outside air is allowed to flow when the sealing by the close-fitting part is undone.

### Advantageous Effects of Invention

According to the present invention, the seal member includes the close-fitting part configured to seal the collecting part by closely fitting on the outer peripheral surface of the dialysate extraction port, and the projection projecting toward the inside of the collecting part and having the outer peripheral surface that faces the inner peripheral surface of the collecting part with a gap in between. The projection defines the inflow route into which outside air is allowed to flow when the sealing by the close-fitting part is undone. Such a configuration assuredly prevents the scattering of fluid that may occur when the cap member is detached from the collecting part. Brief Description of Drawings

[Fig. 1] Fig. 1 schematically illustrates a blood purification apparatus to which a dialysate extraction device according to the present invention is applied.
[Fig. 2] Fig. 2 is a perspective view of a dialysate extraction device according to an embodiment of the present invention.
[Fig. 3] Fig. 3 is a three-view drawing of the dialysate extraction device.
[Fig. 4] Fig. 4 illustrates a section taken along line IV-IV given in Fig. 3.
[Fig. 5] Fig. 5 illustrates a section taken along line V-V given in Fig. 3.
[Fig. 6] Fig. 6 illustrates a section taken along line VI-VI given in Fig. 3.
[Fig. 7] Fig. 7 illustrates a section taken along line VII-VII given in Fig. 3.
[Fig. 8] Fig. 8 is an exploded perspective view of the dialysate extraction device, illustrating a collecting part of a dialysate extraction port, a cap member, and a seal member that are seen from above.
[Fig. 9] Fig. 9 is an exploded perspective view of the dialysate extraction device, illustrating the collecting part of the dialysate extraction port, the cap member, and the seal member that are seen from below.
[Fig. 10] Fig. 10 is a five-view drawing of the seal member included in the dialysate extraction device.
[Fig. 11] Fig. 11 is a five-view drawing of the cap member included in the dialysate extraction device.
[Fig. 12] Fig. 12 schematically illustrates how fluid flows in the dialysate extraction device.
[Fig. 13] Fig. 13 schematically illustrates a state where sealing by a close-fitting part of the seal member included in the dialysate extraction device is undone.
[Fig. 14] Fig. 14 schematically illustrates the dialysate extraction device attached to a dialysate flow route provided in the blood purification apparatus (with the collecting part closed).
[Fig. 15] Fig. 15 schematically illustrates the dialysate extraction device attached to the dialysate flow route provided in the blood purification apparatus (with the collecting part closed).
[Fig. 16] Fig. 16 schematically illustrates the dialysate extraction device attached to the dialysate flow route provided in the blood purification apparatus (with the collecting part open).
[Fig. 17] Fig. 17 schematically illustrates the blood purification apparatus to which the dialysate extraction device according to the present invention is applied (with a pressurizing pump generating a negative pressure).
[Fig. 18] Fig. 18 schematically illustrates the blood purification apparatus to which the dialysate extraction device according to the present invention is applied (with an ultrafiltration pump generating a negative pressure).
[Fig. 19] Fig. 19 schematically illustrates the blood purification apparatus to which the dialysate extraction device according to the present invention is applied (with a duplex pump generating a negative pressure). Description of Embodiments

Embodiments of the present invention will now be described specifically with reference to the drawings.

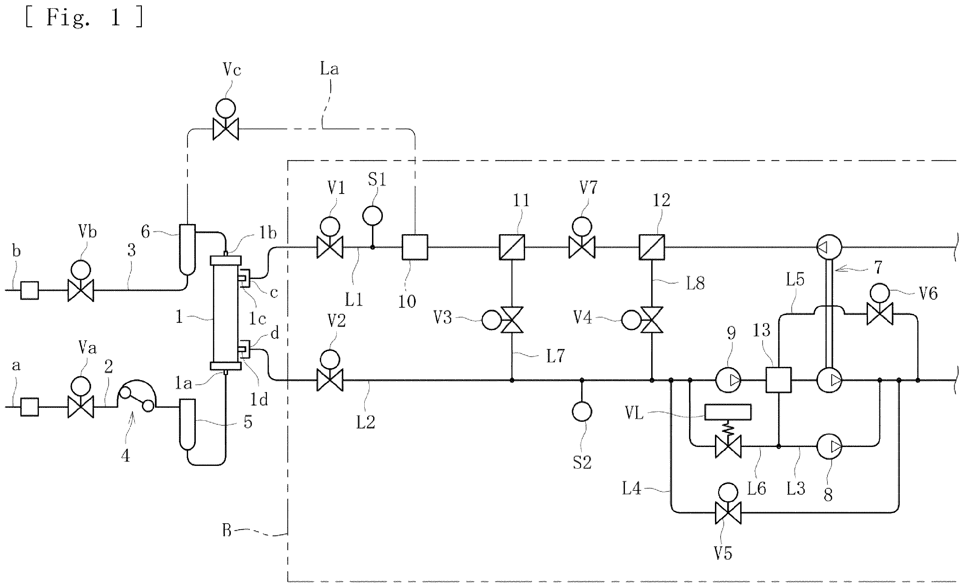

A dialysate extraction device according to an embodiment is provided to a blood purification apparatus intended for blood purification treatment (hemodialysis treatment) and is capable of collecting dialysate. As illustrated in Fig. 1, the applicable blood purification apparatus basically includes a blood circuit, in which an arterial blood circuit 2 and a venous blood circuit 3 are connected to a dialyzer 1 (a blood purifier); and a dialysis device B, which includes a dialysate introduction line L1 and a dialysate discharge line L2.

The dialyzer 1 is intended to purify blood and is connected to the arterial blood circuit 2 and the venous blood circuit 3, included in the blood circuit, at respective ports 1a and 1b. The dialyzer 1 is also connected to the dialysate introduction line L1 and the dialysate discharge line L2 at respective ports 1c and 1d. The arterial blood circuit 2 is provided with a blood pump 4, which is a peristaltic pump. Activating the blood pump 4 enables fluid such as blood to be delivered to flow through the blood circuit.

An arterial puncture needle a and a venous puncture needle b are attachable to the distal end of the arterial blood circuit 2 and the distal end of the venous blood circuit 3, respectively. When the blood pump 4 is activated while a patient is punctured with the arterial puncture needle a and the venous puncture needle b, the patient's blood is collected through the arterial puncture needle a and is caused to extracorporeally circulate through the blood circuit. After the blood is purified and ultrafiltered by the dialyzer 1, the blood is returned to the patient through the venous puncture needle b. The arterial blood circuit 2 is provided with an arterial air-trap chamber 5 and, at a distal portion thereof, a clamping device Va. The venous blood circuit 3 is provided with a venous air-trap chamber 6 and, at a distal portion thereof, a clamping device Vb.

The dialysate introduction line L1 and the dialysate discharge line L2 are connected to a duplex pump 7. The duplex pump 7 is configured to cause dialysate, which is prepared to have a predetermined concentration, to be supplied to the dialyzer 1 and to be discharged from the dialyzer 1. Specifically, the duplex pump 7 is connected astride the dialysate introduction line L1 and the dialysate discharge line L2. Activating the duplex pump 7 enables the dialysate to flow in such a manner as to be introduced into the dialyzer 1 through the dialysate introduction line L1 and to be discharged from the dialyzer 1 through the dialysate discharge line L2.

The dialysate introduction line L1 is provided with filters 11 and 12. The dialysate to be introduced into the dialyzer 1 is filterable by the filters 11 and 12. Electromagnetic valves V1 and V7 are capable of closing and opening the flow route at arbitrary timings. The dialysate introduction line L1 is further provided with a pressure detection sensor S1 on the upstream side relative to the electromagnetic valve V1 (between the electromagnetic valve V1 and a dialysate extraction device 10). The pressure detection sensor S1 is capable of detecting the pressure inside the flow route (the fluid pressure in the flow route). The dialysate introduction line L1 is connected to the dialysate discharge line L2 through bypass lines L7 and L8. The bypass lines L7 and L8 are provided with respective electromagnetic valves V3 and V4.

The dialysate discharge line L2 is provided with detour lines L3 and L4, which detour the duplex pump 7. The detour line L3 is provided with an ultrafiltration pump 8. Hence, activating the ultrafiltration pump 8 in the process of causing the patient's blood to extracorporeally circulate through the blood circuit enables the blood flowing in the dialyzer 1 to be ultrafiltered so that water is removed therefrom. The dialysate discharge line L2 is further provided with a pressurizing pump 9 on the upstream side (the left side in the drawing) relative to the duplex pump 7. The pressurizing pump 9 is a cascade pump and is capable of adjusting the fluid pressure in the dialysate discharge line L2 at the duplex pump 7 (a fluid delivery pump). Between the pressurizing pump 9 and the duplex pump 7 is provided a chamber 13, from which a detour line L5 extends.

The dialysate discharge line L2 is further provided with a pressure detection sensor S2 on the downstream side relative to an electromagnetic valve V2 (between the connection to the bypass line L7 and the connection to the bypass line L8). The pressure detection sensor S2 is capable of detecting the pressure inside the flow route (the fluid pressure in the flow route). The dialysate discharge line L2 and the detour lines L4 and L5 branching off therefrom are provided with the electromagnetic valve V2 and electromagnetic valves V5 and V5, respectively. The electromagnetic valves V2, V5, and V6 are capable of closing and opening the flow route at arbitrary timings. The dialysate discharge line L2 is also provided with a detour line L6, which extends from a point between the connection to the detour line L4 and the pressurizing pump 9 and reaches the detour line L3. The detour line L6 is provided with a relief valve VL.

A dialysate supply line La is connected at one end thereof to a collecting part 14a of a dialysate extraction port 14 of the dialysate extraction device 10, and at the other end thereof to the blood circuit (in the present embodiment, to the venous air-trap chamber 6 connected to the venous blood circuit 3), thereby providing a flow route through which the dialysate in the dialysate introduction line L1 is suppliable to the blood circuit. The dialysate supply line La is provided with a clamping device Vc. The clamping device Vc is openable and closable at arbitrary timings. In the present embodiment, the other end of the dialysate supply line La is connected to the venous air-trap chamber 6. Alternatively, the other end of the dialysate supply line La may be connected to another element of the blood circuit (for example, the arterial air-trap chamber 5 connected to the arterial blood circuit 2, or any other element).

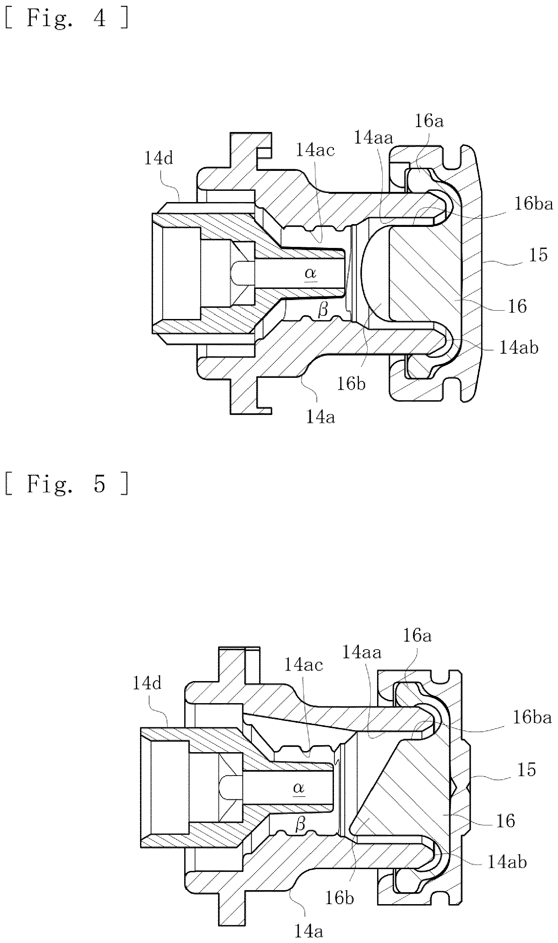

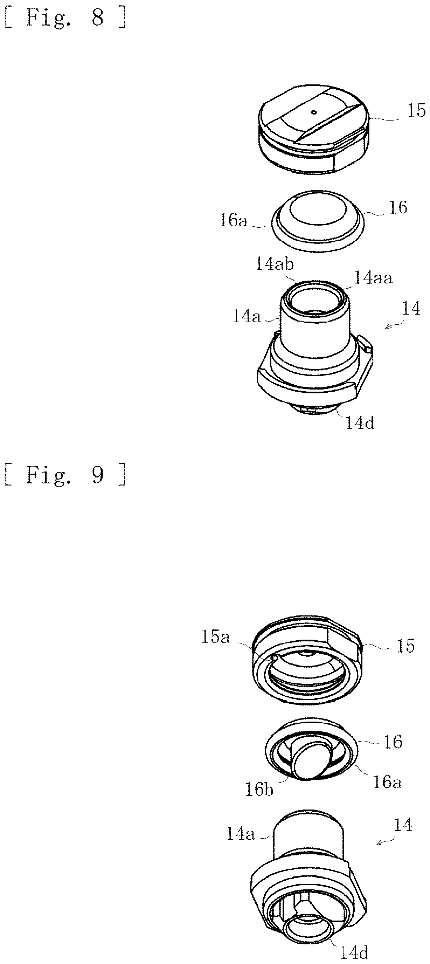

The dialysate introduction line L1 according to the present embodiment is provided with the dialysate extraction device 10, which is capable of collecting the dialysate flowing in the dialysate introduction line L1. As illustrated in Figs. 8, 9, and 14 to 16, the dialysate extraction device 10 includes the dialysate extraction port 14, a cap member 15, and a seal member 16. The dialysate extraction port 14 is connected to the flow route for dialysate (the dialysate introduction line L1) and includes the collecting part 14a, to which the dialysate flowing in the flow route is collectable. The cap member 15 is attachable to and detachable from the collecting part 14a of the dialysate extraction port 14. The seal member 16 is attached to the cap member 15 and is configured to seal the collecting part 14a with the cap member 15 attached to the collecting part 14a. The dialysate extraction device 10 is fixed to the dialysis device B such that, as illustrated in Figs. 4 and 5, the axis (the longitudinal direction) of the dialysate extraction port 14 (the collecting part 14a) extends horizontally. Alternatively, considering the ease of operation, the dialysate extraction device 10 may be fixed to the dialysis device B such that the axis (the longitudinal direction) of the dialysate extraction port 14 (the collecting part 14a) extends at a predetermined angle to the horizontal direction.

The dialysate extraction port 14 includes the collecting part 14a, a first extracting member 14b, a second extracting member 14c, and a third extracting member 14d. The dialysate in the dialysate introduction line L1 is collectable to the collecting part 14a. The first extracting member 14b is connected to the dialysate introduction line L1. The second extracting member 14c is located inside the first extracting member 14b. The third extracting member 14d is fitted on the second extracting member 14c and has a flow route thereinside. As illustrated in Figs. 4 and 5, the collecting part 14a has an inner peripheral surface 14aa and a tip 14ab. A connector (not illustrated) provided at one end of the dialysate supply line La (see Fig. 1) is insertable into the collecting part 14a. The inner peripheral surface 14aa has an internal thread 14ac, along which the connector is screwable.

The first extracting member 14b of the dialysate extraction port 14 according to the present embodiment is provided with an introduction port T1 and an ejection port T2, to which an introduction end L1a and an ejection end L1b of the dialysate introduction line L1 are connected, respectively. A flow route for fluid extends through the second extracting member 14c and the third extracting member 14d and communicates with the introduction port T1. The flow route is provided with a check valve 18. The check valve 18 allows the flow of the fluid from the dialysate supply line La toward the blood circuit but stops the flow of the fluid from the blood circuit toward the dialysate introduction line L1.

The distal end of the third extracting member 14d is located inside the collecting part 14a and is open, so that the fluid flowing in the flow route extending through the second extracting member 14c and the third extracting member 14d reaches the inside of the collecting part 14a. Thus, a first flow route α (see Figs. 4, 5, and 7) is provided for the fluid (such as a cleaning solution or a disinfecting solution) flowing in the dialysate introduction line L1 to flow toward the collecting part 14a. Furthermore, a gap of a predetermined size is provided between the outer peripheral surface of the third extracting member 14d and the inner peripheral surface 14aa of the collecting part 14a. Hence, with the cap member 15 being in the closing state, a second flow route β (see Figs. 4, 5, and 7) is provided for the fluid having flowed through the first flow route α to flow toward the dialysate introduction line L1.

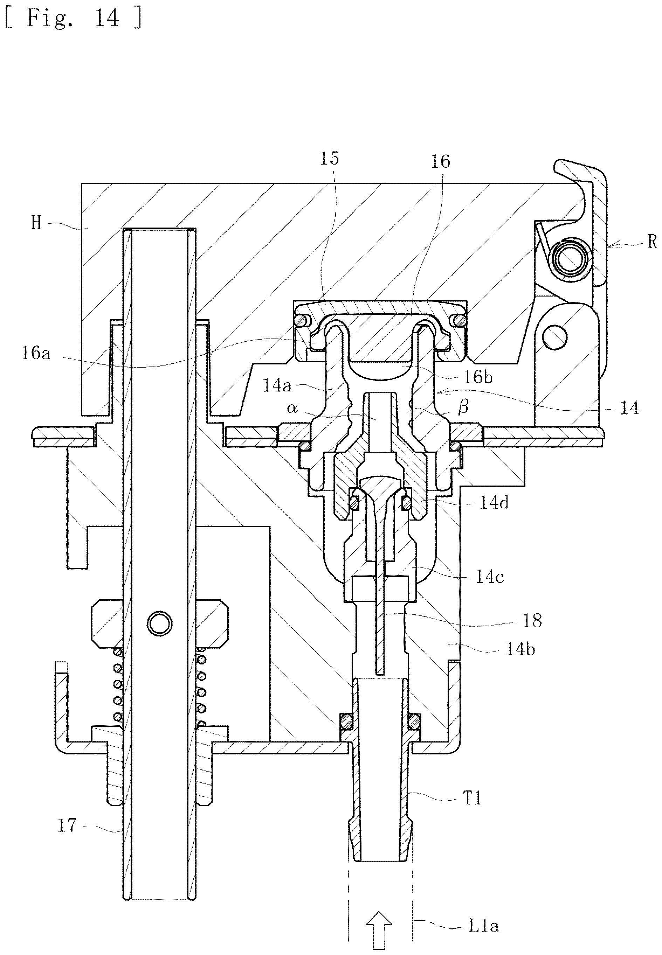

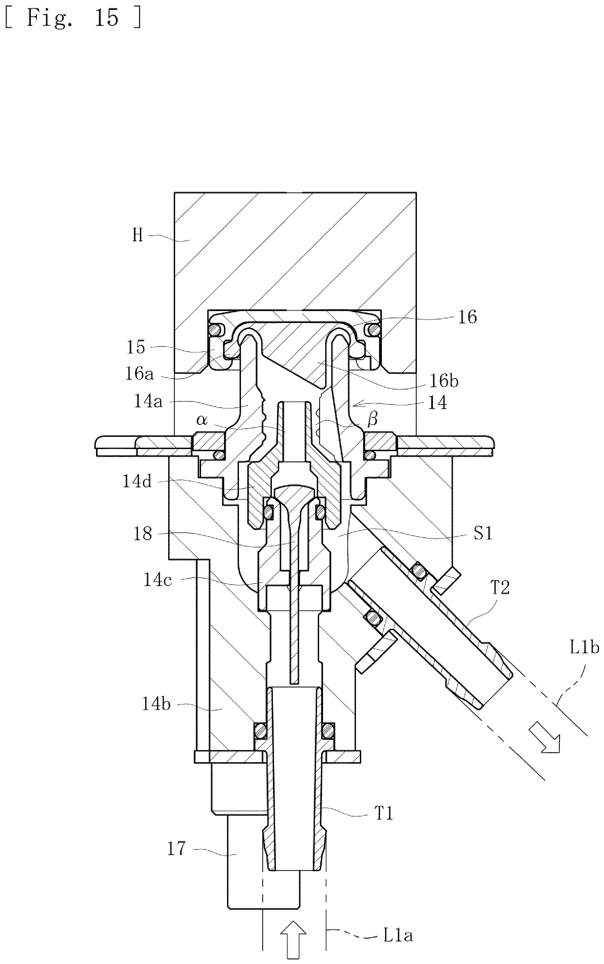

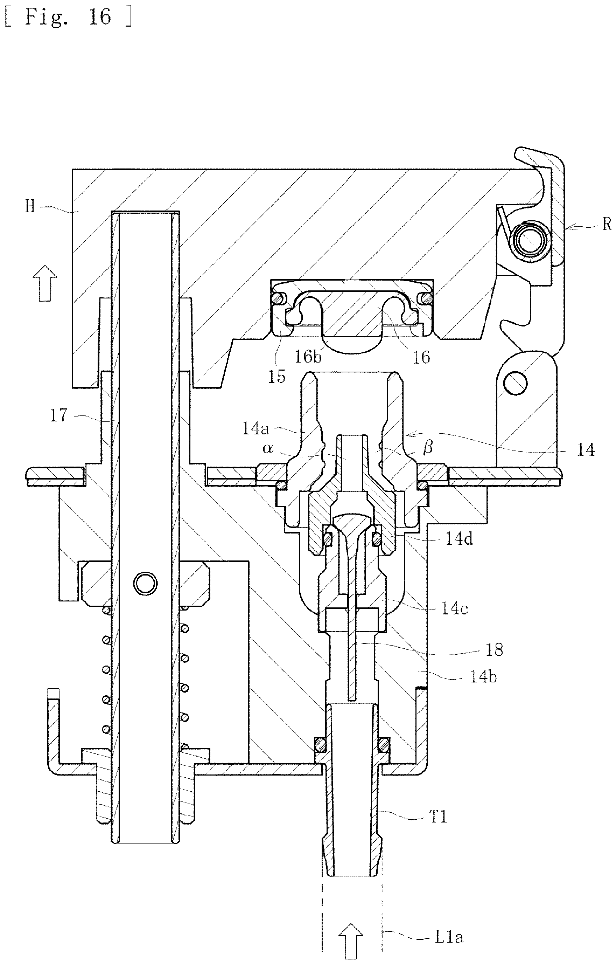

Specifically, with the cap member 15 detached from the dialysate extraction port 14 (see Fig. 16), the dialysate introduced from the introduction port T1 flows through the first flow route α, is discharged from the collecting part 14a, and is supplied into the blood circuit through the dialysate supply line La. In contrast, with the cap member 15 attached to the dialysate extraction port 14 (see Figs. 14 and 15), the dialysate introduced into the first flow route α flows into the second flow route β and returns into the dialysate introduction line L1 through the ejection port T2.

As illustrated in Figs 14 to 16, the cap member 15 according to the present embodiment is held by a lid member H. The lid member H is attached to a shaft member 17, which is movable in the top-bottom direction. When the lid member H is lifted up, as illustrated in Fig. 16, the cap member 15 is detached from the collecting part 14a of the dialysate extraction port 14. When the lid member H is pulled down, as illustrated in Figs. 14 and 15, the cap member 15 is attached to the collecting part 14a of the dialysate extraction port 14. Reference numeral R in the drawings denotes a locking member, which locks the lid member H with the cap member 15 attached to the dialysate extraction port 14.

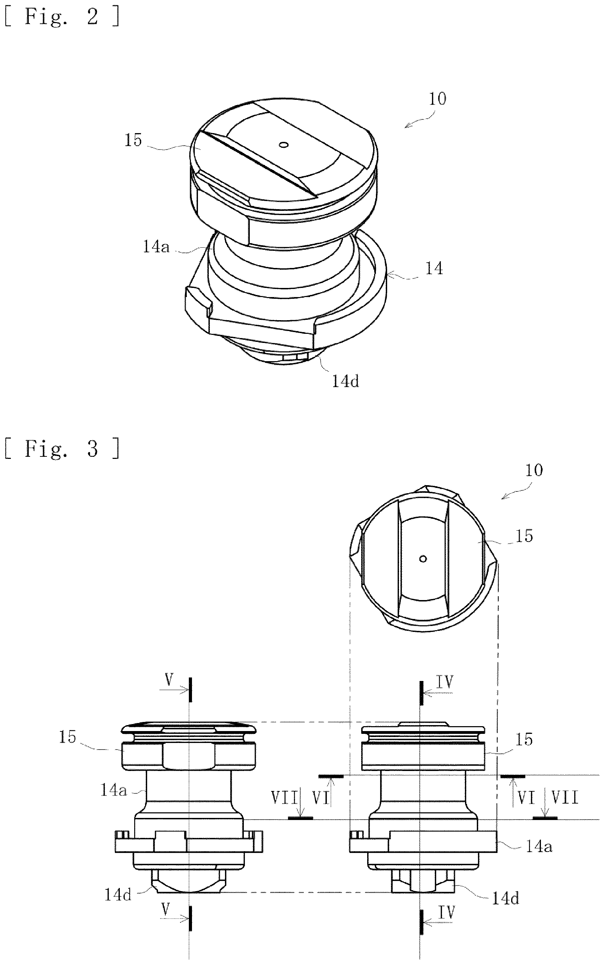

The seal member 16 is made of a flexible material such as a resin material or a rubber material and includes a close-fitting part 16a. As illustrated in Figs. 2 to 7, the seal member 16 is integrated with the cap member 15 by being fitted in a space provided in the cap member 15. With the cap member 15 attached to the collecting part 14a, the seal member 16 covers and seals the opening of the collecting part 14a. The close-fitting part 16a closely fits on the outer peripheral surface of the collecting part 14a of the dialysate extraction port 14, thereby sealing (tightly closing) the collecting part 14a.

With the cap member 15 attached to the collecting part 14a, the seal member 16 prevents the dialysate flowing in the dialysate introduction line L1 from leaking to the outside and allows the fluid, such as a cleaning solution or a disinfecting solution, flowing in the dialysate introduction line L1 to reach the tip 14ab of the collecting part 14a. Such a configuration enables assured cleaning or disinfection of the tip 14ab and a part therearound (including a part facing a space produced between the close-fitting part 16a and the tip 14ab).

As illustrated in Figs. 4 to 6, the seal member 16 according to the present embodiment includes a projection 16b, which projects toward the inside of the collecting part 14a. The projection 16b has an outer peripheral surface 16ba, which faces the inner peripheral surface 14aa of the collecting part 14a with a gap in between. Thus, the projection 16b defines an inflow route γ. When the sealing by the close-fitting part 16a is undone, outside air is allowed to flow into the inflow route γ.

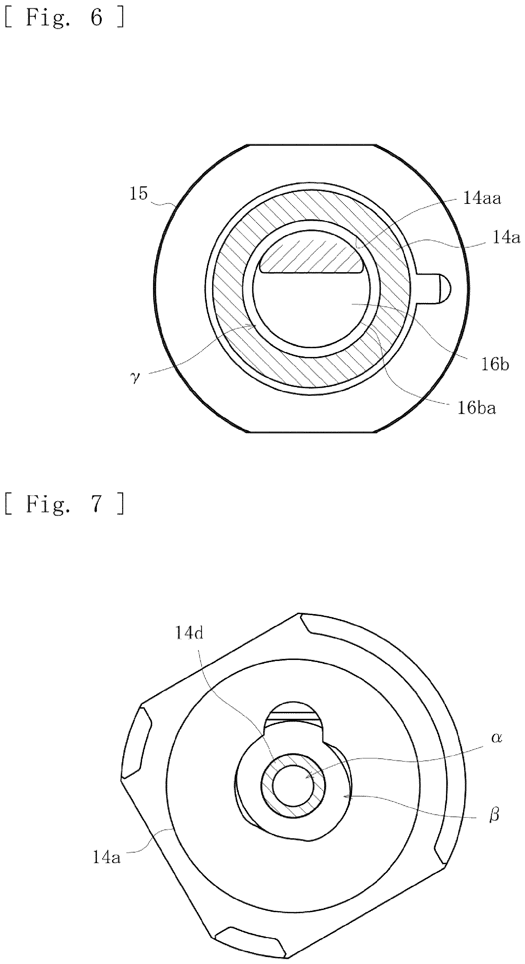

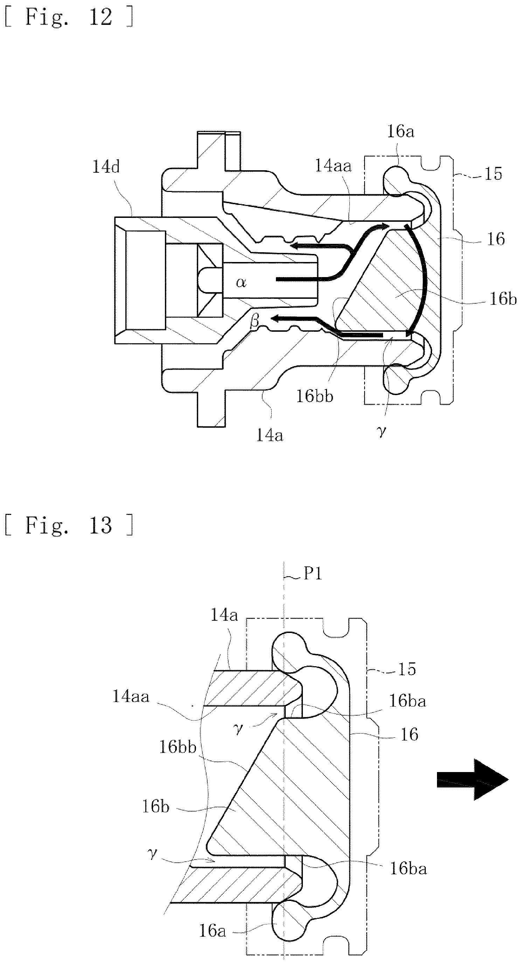

More specifically, as illustrated in Fig. 13, the projection 16b according to the present embodiment has a tip 16bb, which is to be located on the inner side (the left side in Fig. 13) of the collecting part 14a relative to a position P1. The position P1 is reached by the close-fitting part 16a when the sealing of the collecting part 14a is undone. Thus, the inflow route γ is kept secured when the sealing is undone. As illustrated in Fig. 6, the inflow route γ extends over the entire periphery of the outer peripheral surface 16ba of the projection 16b.

Therefore, in the process of detaching the cap member 15 from the collecting part 14a, when the close-fitting part 16a reaches the position P1 and the sealing is undone, outside air flows through the inflow route γ into the collecting part 14a at a high flow speed. Hence, any fluid adhered to and around the tip 14ab of the collecting part 14a is prevented from leaking to the outside. That is, in the process of detaching the cap member 15 from the collecting part 14a, the volume inside the collecting part 14a gradually increases, and the inner pressure decreases. Therefore, when the close-fitting part 16a reaches the position P1 and the sealing is undone, outside air flows into the inflow route γ with a great momentum and flows into the collecting part 14a together with any fluid adhered to the tip 14ab of the collecting part 14a.

As illustrated in Fig. 12, the projection 16b according to the present embodiment is shaped asymmetrically in the top-bottom direction with a lower portion thereof having a greater length of projection than an upper portion thereof. Specifically, in the present embodiment, the dialysate extraction device 10 is fixed such that the axis (the longitudinal direction) of the dialysate extraction port 14 (the collecting part 14a) extends horizontally. In a side view of the projection 16b, the lower portion has a greater length of projection than the upper portion.

Accordingly, the tip 16bb of the projection 16b has a surface inclined downward. Therefore, the fluid, such as a cleaning solution or a disinfecting solution, discharged through the first flow route α toward the projection 16b flows obliquely upward along the tip 16bb to an upper portion of the inflow route γ and then flows in the peripheral direction along the outer peripheral surface 16ba of the projection 16b. The fluid, such as a cleaning solution or a disinfecting solution, having flowed in the peripheral direction along the outer peripheral surface 16ba of the projection 16b flows into a lower portion of the inflow route γ, reaches the second flow route β, and flows into the dialysate introduction line L1.

The fluid adhered to the projection 16b accumulates on the lower side of the projection 16b under its own weight. Considering such a situation, the length of projection of the projection 16b according to the present embodiment is made greater in the lower portion than in the upper portion. That is, the length of the inflow route γ is made greater on the lower side of the projection 16b. Such a configuration causes the fluid accumulated on the lower side of the projection 16b under its own weight to flow inward of the collecting part 14a together with outside air.

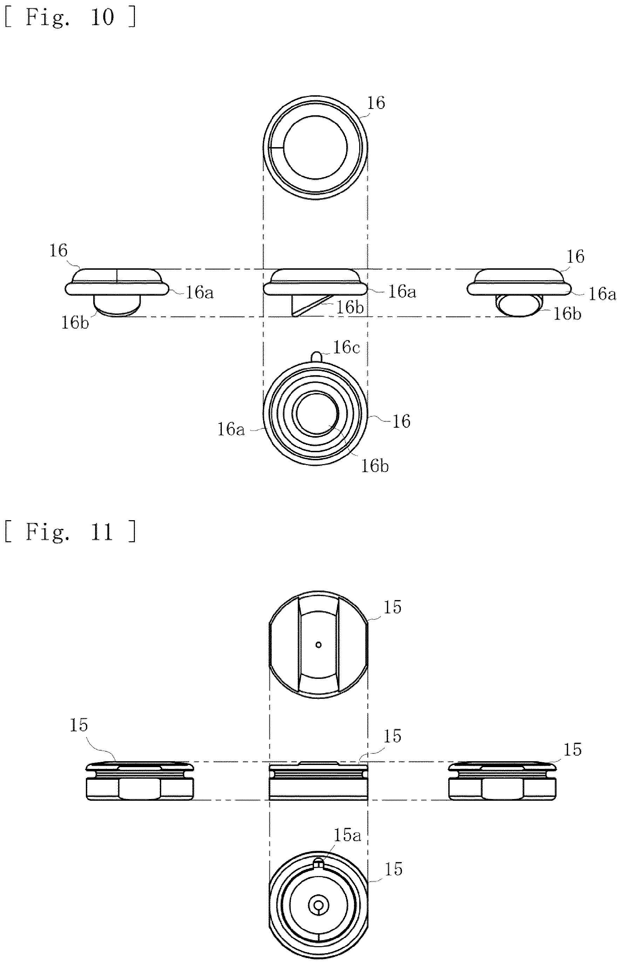

As illustrated in Fig. 11, the cap member 15 according to the present embodiment includes a positioning part 15a, where the seal member 16 is positionable. Specifically, the cap member 15 has the positioning part 15a, which is a cut provided at a predetermined position. On the other hand, as illustrated in Fig. 10, the seal member 16 has a protrusion 16c, which is fittable into the positioning part 15a. The seal member 16 is attached to the cap member 15 such that the protrusion 16c is fitted into the positioning part 15a. Thus the seal member 16 is positionable.

The blood purification apparatus applicable to the present embodiment is configured to generate a negative pressure in the dialysate extraction port 14 at a predetermined timing before the cap member 15 is detached from the collecting part 14a. A device for generating a negative pressure in the dialysate extraction port 14 may be a fluid delivery unit configured to deliver the dialysate in the dialysate introduction line L1 (the dialysate flow route). In the present embodiment, the pressurizing pump 9, which is a cascade pump, is activated to generate a negative pressure in the dialysate extraction port 14.

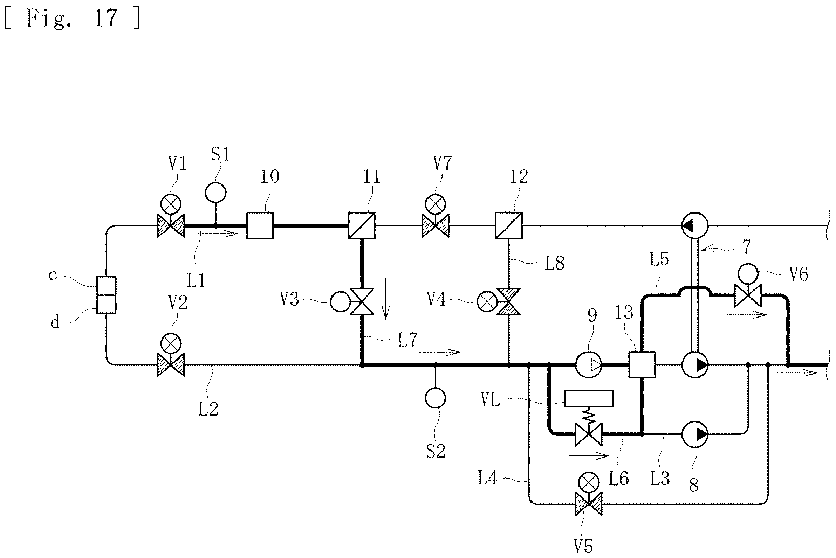

Now, how to generate a negative pressure in the dialysate extraction port 14 by activating the pressurizing pump 9 will be described with reference to Fig. 17.

First, the distal end c of the dialysate introduction line L1 and the distal end d of the dialysate discharge line L2 are connected to each other. Furthermore, the electromagnetic valves V1, V2, V4, V5, and V7 are closed (the flow route is closed), whereas the electromagnetic valves V3 and V6 are opened (the flow route is opened). Then, the pressurizing pump 9 is activated. In this state, the ultrafiltration pump 8 and the duplex pump 7 are not in operation. Thus, the volume of the dialysate in a portion of the dialysate introduction line L1 where the dialysate extraction device 10 is connected is reduced. Accordingly, a negative pressure is generated in the dialysate extraction port 14.

Subsequently, if the electromagnetic valves V1, V3, and V7 are closed, the negative pressure generated in the dialysate extraction device 10 can be retained. In this state, for example, if information that the negative pressure is retained is provided on a display monitor or the like, the detaching of the cap member 15 from the collecting part 14a can be prompted. If the electromagnetic valve V4 is opened with the negative pressure in the dialysate extraction device 10 retained, activating the duplex pump 7 can cause the dialysate to flow through the bypass line L8.

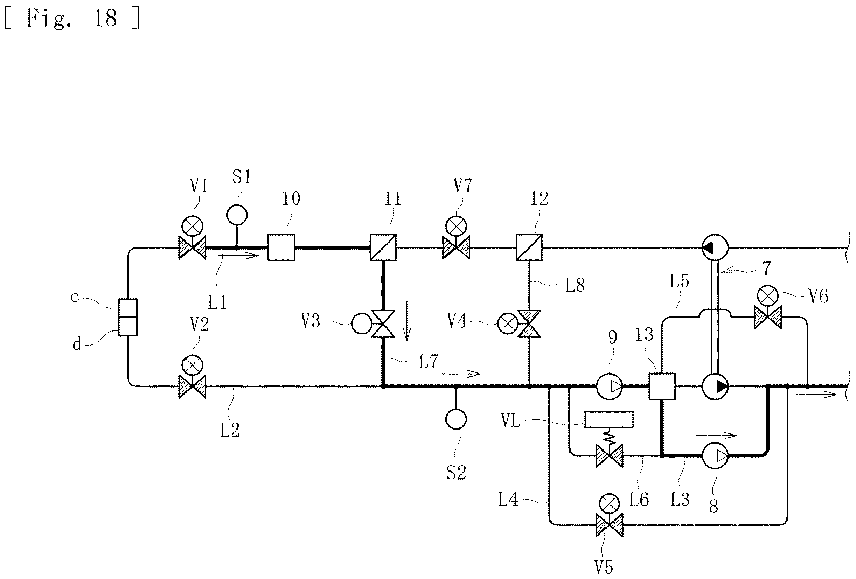

The negative pressure in the dialysate extraction port 14 may alternatively be generated by activating the ultrafiltration pump 8. In that case, as illustrated in Fig. 18, the distal end c of the dialysate introduction line L1 and the distal end d of the dialysate discharge line L2 are connected to each other. Furthermore, the electromagnetic valves V1, V2, V4, V5, V6, and V7 are closed (the flow route is closed), whereas the electromagnetic valve V3 is opened (the flow route is opened). Then, the ultrafiltration pump 8 is activated. Thus, the volume of the dialysate in the portion of the dialysate introduction line L1 where the dialysate extraction device 10 is connected is reduced. Accordingly, a negative pressure is generated in the dialysate extraction port 14. In this state, if the electromagnetic valves V1, V3, and V7 are closed, the negative pressure generated in the dialysate extraction device 10 can be retained.

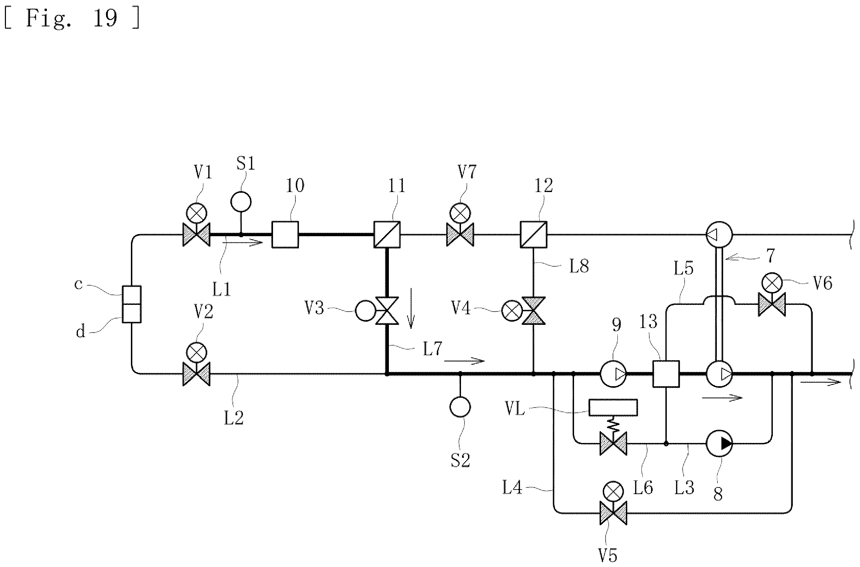

As another alternative, the negative pressure in the dialysate extraction port 14 may be generated by activating the duplex pump 7. In that case, as illustrated in Fig. 19, the distal end c of the dialysate introduction line L1 and the distal end d of the dialysate discharge line L2 are connected to each other. Furthermore, the electromagnetic valves V1, V2, V4, V5, V6, and V7 are closed (the flow route is closed), whereas the electromagnetic valve V3 is opened (the flow route is opened). Then, the duplex pump 7 is activated. Thus, the volume of the dialysate in the portion of the dialysate introduction line L1 where the dialysate extraction device 10 is connected is reduced. Accordingly, a negative pressure is generated in the dialysate extraction port 14. In this state, if the electromagnetic valves V1, V3, and V7 are closed, the negative pressure generated in the dialysate extraction device 10 can be retained.

According to the present embodiment, the seal member 16 includes the close-fitting part 16a configured to seal the collecting part 14a by closely fitting on the outer peripheral surface of the dialysate extraction port 14, and the projection 16b projecting toward the inside of the collecting part 14a and having the outer peripheral surface 16ba that faces the inner peripheral surface 14aa of the collecting part 14a with a gap in between. The projection 16b defines the inflow route γ into which outside air is allowed to flow when the sealing by the close-fitting part 16a is undone. Such a configuration assuredly prevents the scattering of the fluid that may occur when the cap member 15 is detached from the collecting part 14a.

According to the present embodiment, the projection 16b has the tip 16bb to be located on the inner side of the collecting part 14a relative to the position P1 that is reached by the close-fitting part 16a when the sealing of the collecting part 14a is undone. Furthermore, the inflow route γ is kept secured when the sealing is undone. Therefore, when the sealing of the collecting part 14a is undone, the inflow route γ is kept secured without fail. Such a configuration more assuredly prevents the scattering of the fluid that may occur when the cap member 15 is detached from the collecting part 14a.

The inflow route γ extends over the entire periphery of the outer peripheral surface 16ba of the projection 16b. Such a configuration causes any fluid adhered to and around the tip 14ab of the collecting part 14a to flow inward of the collecting part 14a, together with air, in an area along the entire periphery. The dialysate extraction port 14 has the first flow route α through which the fluid flowing in the dialysate introduction line L1 is allowed to flow toward the collecting part 14a, and the second flow route β through which the fluid having flowed through the first flow route α is allowed to flow toward the dialysate introduction line L1. Furthermore, the projection 16b is shaped asymmetrically in the top-bottom direction with the lower portion thereof having a greater length of projection than the upper portion thereof. Such a configuration smoothly causes the fluid, such as a cleaning solution or a disinfecting solution, having flowed through the first flow route α and discharged toward the projection 16b to flow from the second flow route β to the dialysate introduction line L1.

The cap member 15 includes the positioning part 15a where the seal member 16 is positionable. Such a configuration enables the projection 16b shaped asymmetrically in the top-bottom direction to be assuredly oriented in a predetermined way such that a portion thereof having a greater length of projection is located on the lower side while a portion thereof having a smaller length of projection is located on the upper side.

In the blood purification apparatus applied to the present embodiment, a negative pressure is generated in the dialysate extraction port 14 at a predetermined timing before the cap member 15 is detached from the collecting part 14a. Such a configuration assuredly prevents the scattering of the fluid that may occur when the cap member 15 is detached from the collecting part 14a. According to the present embodiment, the negative pressure in the dialysate extraction port 14 is generated by activating the fluid delivery unit (the pressurizing pump 9, the ultrafiltration pump 8, or the duplex pump 7) configured to deliver the dialysate in the dialysate introduction line L1. Thus, a component necessary for blood purification treatment is utilizable. In particular, if the fluid delivery unit to be activated is the pressurizing pump 9 that is a cascade pump, the volume is gradually reduced by a constant volume without pulsation, realizing a smooth generation of a negative pressure.

While some embodiments have been described above, the present invention is not limited thereto. For example, the projection 16b does not necessarily need to be shaped asymmetrically in the top-bottom direction, as long as the projection 16b defines the inflow route γ into which outside air is allowed to flow when the sealing by the close-fitting part 16a is undone. Specifically, the projection 16b may be a projection having a cylindrical shape (whether hollow or solid). The applicable blood purification apparatus is not limited to the one configured to generate a negative pressure in the dialysate extraction port 14 at a predetermined timing before the cap member 15 is detached from the collecting part 14a. The blood purification apparatus does not necessarily need to have such a function.

The blood purification apparatus to which any of the above embodiments is applied may be provided in any mode. For example, the blood purification apparatus may be any of the following: an apparatus in which the dialysate is to be introduced or discharged by using a chamber instead of the duplex pump 7, an apparatus including a blood purifier in a mode other than the dialyzer 1, and an apparatus including neither the pressure detection device S1 nor the pressure detection device S2. While the above embodiments each concern a case where the dialysate extraction device is provided to the dialysate introduction line L1 provided in the dialysis device, the dialysate extraction device may be provided to another dialysate flow route provided in the dialysis device.

### Industrial Applicability

The present invention is also applicable to any dialysate extraction device or any blood purification apparatus having a different appearance, additional functions, or the like within the spirit of the present invention.

### Reference Signs List

1 dialyzer (blood purifier)
2 arterial blood circuit
3 venous blood circuit
4 blood pump
5 arterial air-trap chamber
6 venous air-trap chamber
7 duplex pump
8 ultrafiltration pump
9 pressurizing pump (cascade pump)
10 dialysate extraction device
11, 12 filter
13 chamber
14 dialysate extraction port
14a collecting part
14aa inner peripheral surface
14ab tip
14ac internal thread
14b first extracting member
14c second extracting member
14d third extracting member
15 cap member
15a positioning part
16 seal member
16a close-fitting part
16b projection
16ba outer peripheral surface
16bb tip
16c protrusion
17 shaft member
18 check valve
L1 dialysate introduction line (dialysate flow route)
L2 dialysate discharge line
B dialysis device
H lid member
S1, S2 pressure detection sensor
α first flow route
β second flow route
γ inflow route

## Claims

1. A dialysate extraction device comprising:
a dialysate extraction port connected to a dialysate flow route through which dialysate is to flow, the dialysate extraction port including a collecting part to which the dialysate flowing in the dialysate flow route is collectable;
a cap member attachable to and detachable from the collecting part of the dialysate extraction port; and
a seal member attached to the cap member and configured to seal the collecting part with the cap member attached to the collecting part,
wherein the seal member includes
a close-fitting part configured to seal the collecting part by closely fitting on an outer peripheral surface of the dialysate extraction port; and
a projection projecting toward an inside of the collecting part and having an outer peripheral surface that faces an inner peripheral surface of the collecting part with a gap in between, the projection defining an inflow route into which outside air is allowed to flow when the sealing by the close-fitting part is undone.

2. The dialysate extraction device according to Claim 1, wherein the projection has a tip to be located on an inner side of the collecting part relative to a position that is reached by the close-fitting part when the sealing of the collecting part is undone, and the inflow route is kept secured when the sealing is undone.

3. The dialysate extraction device according to Claim 1 or 2, wherein the inflow route extends over an entire periphery of the outer peripheral surface of the projection.

4. The dialysate extraction device according to Claim 3, wherein the dialysate extraction port has a first flow route through which fluid flowing in the dialysate flow route is allowed to flow toward the collecting part; and a second flow route through which the fluid having flowed through the first flow route is allowed to flow toward the dialysate flow route, and wherein the projection is shaped asymmetrically in a top-bottom direction with a lower portion of the projection having a greater length of projection than an upper portion of the projection.

5. The dialysate extraction device according to Claim 4, wherein the cap member includes a positioning part where the seal member is positionable.

6. A blood purification apparatus comprising the dialysate flow route to which the dialysate extraction device according to any of Claims 1 to 5 is attached,
wherein a negative pressure is generated in the dialysate extraction port at a predetermined timing before the cap member is detached from the collecting part.

7. The blood purification apparatus according to Claim 6, further comprising a fluid delivery unit configured to deliver dialysate in the dialysate flow route, wherein the negative pressure in the dialysate extraction port is generated by activating the fluid delivery unit.

8. The blood purification apparatus according to Claim 7, wherein the fluid delivery unit is a cascade pump.
